# EUROPEAN PATENT SPECIFICATION

(11) **EP 1 033 976 B1**
(45) Date of publication and mention of the grant of the patent: **23.08.2006**
(21) Application number: 97945962.5
(22) Date of filing: 27.11.1997
(51) Int. Cl.: A61K 9/16

(54) **MICROSPHERE RESERVOIRS FOR CONTROLLED RELEASE APPLICATION**
BEHÄLTER IN FORM VON MIKROSPHÄREN ZUR GESTEUERTEN FREISETZUNG
RESERVOIRS EN MICROSPHERES POUR APPLICATIONS A LIBERATION CONTROLEE

(43) Date of publication of application: 13.09.2000
(73) Proprietor: Arch UK Biocides Limited, Castleford, West Yorkshire WF10 2JT (GB)
(72) Inventor: LO, Ray, Jiaruey, San Leandro, CA 94579 (US)
(74) Representative: Bannerman, David Gardner
(86) International application number: PCT/GB1997/003260
(87) International publication number: WO 1999/027910

(56) References cited:
- US-A- 5 725 869
- PALOMO M E ET AL: "SOLVENT AND PLASTICIZER INFLUENCES ON ETHYLCELLULOSE -MICROCAPSULES" JOURNAL OF MICROENCAPSULATION, vol. 13, no. 3, 1 May 1996, pages 307-318, XP000583514
- TEFFT J ET AL: "CONTROLLED RELEASE HERBICIDE FORMULATIONS BASED ON POLYMERIC MICROSPHERES" JOURNAL OF CONTROLLED RELEASE, vol. 27, no. 1, 1 October 1993, pages 27-35, XP000397038

## Description

This invention relates to controlled-release compositions in the form of microspheres containing one or more ingredients to be dispensed at a controlled rate, and to the microspheres themselves. Controlled-release formulations are used in a number of industries and for a number of types of products such as agricultural products (fertilisers, pesticides, soil nutrients, etc.) drugs and pharmaceuticals, fragrances and cosmetics. Controlled-release formulations permit the application of a product containing a relatively concentrated amount of an ingredient which is to be dispensed (sometimes referred to as the "active ingredient"), which is then applied or dispensed relatively slowly and over a relatively long period of time. This permits, for instance, the application of a pesticide or a pharmaceutical over a long period of time to effect a steady treatment as opposed to a quick release of the ingredient, which may result in initially supplying more material than is needed, or having an insufficient amount of material available at a later time. The use of controlled-release formulations also permits the application and handling of materials containing a higher concentration of active ingredient while minimising the possible toxicity consequences of such concentration, since the exposure to the active ingredient is relatively limited in amount at a given time.

There are a number of different types of controlled-release formulations. One such type involves encasing or encapsulating the active ingredient in microspheres. In agriculture, microspheres containing active ingredients may be produced which are dispersible in water so as to be used in conventional spraying equipment.

One technique for producing microspheres is known as the "solvent evaporation" technique or process. In that type of process, an active ingredient is dissolved in a solvent, along with additives and is mixed with an aqueous solution containing a polymer which will form an encapsulating spherical wall. The mixture is in the form of an oil-in-water emulsion, with the organic phase in the form of droplets of a polymer surrounding a solution of the active ingredient. The solvent (together with the water) is then evaporated to form microspheres. A typical process of this type is described in the paper by Tefft, et al, Proc. Int. Symp. Control. Rel. Bioact. Mater., page 19 (1992), entitled "Microspheres for Controlled-Release Herbicide Formulations". That article describes preparation of microspheres composed of ethyl cellulose or polyarylsulfone polymer including the herbicide dicamba as the active ingredient, by dissolving dicamba and the polymer in methylene chloride, emulsifying that solution into an aqueous solution containing polyvinyl alcohol and sodium dodecyl sulphate, stirring and evaporating the solvent under vacuum.

It has been found, however, that production of microspheres by the solvent evaporation method can result in microspheres which, while permitting controlled release, nevertheless permit too rapid a release of the active ingredient. An overly rapid release may be undesirable in a given situation for the reasons mentioned above, for instance it provides an overdose of, or wastes, the active ingredient.

This invention comprises the production of microspheres of an ingredient to be dispensed by the solvent evaporation method in which a plasticiser selected from the group consisting of phthalate esters, phosphate esters, citrate esters, sebacate esters, glycerol, triacetin and acetylated monoglyceride, is included.

In another aspect, this invention comprises a process for producing microspheres containing an ingredient to be dispensed comprising:
(a) Preparing an organic phase comprising a solution of an ingredient to be dispensed, a polymer and a plasticiser selected from the group consisting of phthalate esters, phosphate esters, citrate esters, sebacate esters, glycerol, triacetin or acetylated monoglyceride, in an organic solvent;
(b) Preparing an aqueous phase comprising an aqueous solution of an emulsifying agent;
(c) Combining the organic and aqueous phases under emulsifying conditions to form an emulsion of the organic phase in the aqueous phase; and
(d) Evaporating the solvent to form microspheres comprising the polymer, the plasticiser and the ingredient to be dispensed.

In yet another aspect, this invention comprises a process for production of microspheres which are "blank", i.e., do not contain an ingredient to be dispensed. Such blank microspheres can be loaded at a subsequent time with one or more active ingredients.

The invention herein comprises both the processes mentioned above as well as the products of said processes, blank microspheres or microspheres containing the ingredient to be dispensed.

Production of the microspheres of this invention is carried out using the solvent-evaporation technique, generally described above, but with the additional inclusion of a plasticiser.

In this process, two solutions are prepared: An organic solution containing a polymer, a plasticiser and (when the microspheres are to be produced "loaded") material to be dispensed ("active ingredient"), and an aqueous solution containing an emulsifying agent. This solution may contain polyvinyl alcohol, which may serve as an emulsifying agent and/or to control viscosity. The two solutions are then combined under conditions so as to form an emulsion of the organic phase in the aqueous phase, which is then converted to an encapsulated product comprising microspheres composed of a polymer and plasticising agent (and containing the ingredient to be dispensed), by evaporating the solvent, either under heat or vacuum.

The polymer utilised in the process according to this invention may be selected from a wide number of types known to be useful in producing controlled-release formulations. These include both synthetic polymers as well as naturally-occurring polymers and their derivatives.

Synthetic polymers include, for instance, vinyl polymers, polyamides, polyureas, polyurethanes, polyesters, acrylates, methacrylates, polyarylsulfone, acrylate-methacrylate copolymers, and poly(ethylene oxide) polymeric resins such as those sold under the trade mark POLYVOX.

Naturally-occurring polymers and their derivatives include materials such as cellulosic materials, starches, lignins, vegetable gums, alginates, and derivatives thereof such as polysaccharides, proteins, shellac, and resins. The polymer may also be a blend of one or more of the above synthetic or naturally-occurring materials.

The plasticiser utilised in the process of this invention is selected from phthalate esters, phosphate esters, citrate esters, sebacate esters, glycerole, triacetin, and acetylated monoglyceride. Examples of plasticisers of the above types include dimethyl, diethyl, dipropyl, dibutyl, dibutoxyethyl and dioctyl phthalates; tricresyl and triphenyl phosphates; triethyl, tributyl, and acetyl tributyl citrates; and dibutyl sebacate.

Preferred polymers for use in the process according to this invention are cellulosic polymers and derivatives thereof. Preferred plasticisers are phthalate esters.

The choice of plasticiser for use with a given polymer is within the skill of those in the art and, in general, any of the types of plasticisers mentioned above may be combined or used with any of the polymers mentioned above, with the choice of plasticisers being made to achieve optimum effects.

The ingredient to be dispensed may be a chemical or other ingredient for use in agriculture, a pharmaceutical or other medicinal ingredient, a fragrance, a cosmetic, or any other type of ingredient for which a controlled release or dispensing is desirable. Agricultural materials include pesticides (for instance herbicide, insecticides, fungicides, bactericides and the like), plant growth regulators, fertilisers, and soil nutrients. The microspheres may contain more than one ingredient to be dispensed, for instance a combination of two or more pesticides or two or more pharmaceuticals.

Preferred agricultural materials include the pesticides lambda-cyhalothrin [1α(S*), 3α(Z)]-(±)cyano-(3-phenoxybenzyl)-(2-chloro-3,3,3-trifluoro-1-propenyl)-2,2-dimethylcyclopropane carboxylate, paraquat [1,1'-dimethyl-4,4'-bipyridinium dichloride or other salts], napropramide (N,N-diethyl-2-(α-naphthoxy)propionamide) and fluazifop-butyl {butyl (R or RS)-2-[4-[[5-(trifluoromethyl)-2-pyridinyl]oxy]-phenoxy]propanoate}.

The solvent utilised in the process is chosen from among typical solvents on the basis of three properties. First, the solvent must dissolve the polymer, the plasticiser and the ingredient to be dispensed (if the microspheres are to be produced "loaded"). Secondly, the solvent preferably should have a relatively low boiling point so as to be easily removed through evaporation. Thirdly, the solvent must be water-immiscible. Suitable solvents include hydrocarbon such s pentane, hexane, heptane, cyclohexane and the like, and chlorinated solvents such as dichloromethane.

The emulsifying agent may be any of a number of types of known to cause emulsions to form when organic and aqueous phases are mixed. Typical emulsifying agents include surfactants such as polyethylene glycol ethers of linear alcohols, ethoxylated nonylphenols, naphthalene sulphonates, ethylene oxide/propylene oxide block copolymers, alkali metal/alkyl sulphates and other salts, and the like.

The organic phase will typically contain from 1 to 50 weight per cent polymer and from 0.1 to 20 weight per cent plasticiser, depending on the nature of the polymer, plasticiser and solvent. The aqueous phase will typically contain from 0.1 to 20 weight per cent of emulsifying agent and optionally from 1 to 30 weight per cent polyvinyl alcohol. The composition of the final microsphere product will be from 5 to 80 weight per cent polymer, from 0.5 to 10 weight per cent of the plasticiser, and up to 70 weight per cent of the ingredient to be dispensed. The microspheres will be from 3 to 300 microns in diameter.

Production of microspheres using cellulosic polymers (for example ethyl cellulose) by the solvent-evaporation method is not new. For instance, production of very small microspheres (0.2-1 microns diameter) from plasticised ethyl cellulose polymers is described by Ghebre-Sellassie et al., Pharmaceutical Technology, page 96 (September 1998). Such microspheres, however, were produced for use in a controlled-release film to be coated over an ingredient to be dispensed rather than to contain the ingredient. Additionally, such microspheres, which are approximately 0.2-1 micron diameter are much smaller than those of the present invention (between 3 and 300 microns diameter).

The Tefft et al. work, on the other hand, does produce microspheres of sizes similar to those produced by the present invention, and which did contain an ingredient to be dispensed (dicamba herbicide). However, it was found that the spheres produced by Tefft et al., who did not use a plasticiser, were in the shape of thin-walled hollow spheres containing dicamba whereas those produced by the present process are not hollow spheres, but have a spongy structure which may permit the ingredient to be released at a slower rate. Advantages of such a slower dispensing rate include lower toxicity of material to handler, and a more controlled use of ingredient.

Because of their spongy structure, the microspheres of this invention (as opposed to the hollow spheres of Tefft et al.) may be produced "blank", that is without containing an ingredient to be dispensed. Such ingredient may be added at a later date, for instance by soaking or dipping the micrspheres in a solution of the ingredient, spraying the ingredient on the spheres or mixing the ingredient and spheres in a mixer. Blank microspheres are produced by carrying out the process as described herein except that an ingredient to be dispensed is not included.

The production of blank microspheres to be loaded later will be advantageous in several situations. For example, if long-term storage of encapsulated products is likely to occur, the active ingredient could begin to migrate out of the capsules during storage. Production of blank capsules would permit separate long-term storage of the capsules and the active ingredient, with the vinyl "loaded" product produced relatively shortly before expected use. Also, production of blank microspheres may be advantageous in a multi-product plant in which microspheres are to be loaded with different ingredients to be dispensed.

The size of the microspheres depends to a great extend on the speed with which the emulsion is stirred. The higher the stirring speed, the smaller the droplet of active ingredient plus polymer formed in the aqueous solution. Preferred stirring speeds are 500-2,500 rpm. The resulting microspheres form a dry, free-flowing powder which can have a high loading of the active ingredient or ingredient to be dispensed. Furthermore, the process for the production can be carried out at room temperature and does not require elevated temperatures or expensive capital equipment.

The following represents examples of the production of microspheres according to this invention.

### General Procedure

Ethyl cellulose (ethoxyl content 48%), was dissolved in dichloromethane to produce a 3.5% weight/volume polymer solution. Then, the indicated amount of diethyl phthalate was dissolved into that solution, followed by indicated amounts of the insecticide lambda-cyhalothrin. The amounts of lambda-cyhalothrin were chosen to provide between 100 to 300 weight per cent of that substance relative to the amount of polymer dissolved in the solution.

The solution was then emulsified into an aqueous solution containing 5 weight per cent polyvinyl alcohol and, at times, 0.01 weight per cent sodium dodecyl sulphate. The mixture was then stirred for 15 minutes at room temperature at the indicated speeds; then a mercury vacuum (approximately 25 inch) was applied to remove the dichloromethane solvent. The microspheres were collected by filtration, re-suspended in water containing 0.5 weight per cent sodium dodecyl sulphate, and dried.

### Comparison Examples

Using the same general procedure as above, but omitting the addition of diethyl phthalate, microspheres containing lambda-cyhalothrin were obtained.

The table summarises the results of these experiments.

**TABLE**

| Experiment No. | Lambda-cyahlotrin wt.% | Ethyl cellulose wt.% | Diethyl phthalate wt.% | Stirring speed rpm | Microsphere size microns |
|---|---|---|---|---|---|
| Invention | | | | | |
| 1 | 40 | 40 | 5 | 800 | 65 |
| 2 | 41 | 41 | 5 | 2500 | 8 |

| Comparison (Tefft et al. process) | | | | | |
|---|---|---|---|---|---|
| A | 63 | 22 | - | 500 | 180 (est.) |
| B | 40 | 40 | - | 2500 | 11 |

The microcapsules prepared according to the invention were compared with those prepared according to the comparison example, by scanning electron microscope and regular microscope. It was observed that the microspheres prepared according to this invention were roughly spheroidal with an uneven outer surface and high porosity whereas those prepared according to the comparison example had smooth spherical surfaces with only a few pores visible.

Both types of microspheres were cut by a razor and again viewed. Those produced by the process of this invention were shown to have a highly porous surface and a highly porous internal structure as well, resembling a sponge. On the other hand, those produced in the comparison example appeared to have hollow cores with a number of small holes on the outer shell. This combination of porous, spongy structure makes the microspheres of the invention eminently suitable for applications in which a high loading of material to be dispensed, combined with a slow release rate, is desirable.

## Claims

1. A process for producing porous microspheres having a diameter between 3 and 300 microns comprising:
(a) preparing an organic phase comprising a solution of a polymer and a plasticiser selected from the group consisting of phthalate esters, phosphate esters, citrate ester, sebacate esters, glycerol, triacetin and acetylated monoglycerides, in an organic solvent;
(b) preparing an aqueous phase comprising an aqueous solution of one or more emulsifying agents;
(c) combining the organic and aqueous phases under emulsifying conditions to form an emulsion of the organic phase in the aqueous phase; and
(d) evaporating the solvent to form microspheres.

2. A process as claimed in Claim 1 in which the microspheres further contain an ingredient to be dispensed, said ingredient to be dispensed being contained in the organic phase prepared in step (a).

3. A process as claimed in Claim 2 in which the ingredient to be dispensed is an agricultural chemical, a pharmaceutical, a medicine, a cosmetic or a fragrance.

4. A process as claimed in Claim 3 in which the ingredient to be dispensed is an agricultural chemical.

5. A process as claimed in Claim 2 wherein the ingredient to be dispensed is a fungicide or bactericide.

6. A process as claimed in any one of Claims 1 to 5 in which the polymer is a cellulosic polymer or a derivative thereof.

7. A process as claimed in any one of Claims 1 to 6 in which the plasticiser is a phthalate ester.

8. A process as claimed in any one of Claims 1 to 7 in which the emulsifying agent comprises polyvinyl alcohol.

9. A process as claimed in any one of Claims 1 to 8 in which the polymer is present in the organic phase in an amount of from 1 to 50 weight per cent and the plasticiser is present in the organic phase in an amount of from 0.1 and 20 weight per cent relative to the polymer.

10. A process as claimed in any one of Claims 2 to 9 in which the microspheres contain up to 70 weight per cent of the ingredient to be dispensed.

11. A process as claimed in any one of claims 1 to 10 in which the polymer is contained in the organic phase in an amount of between 1 and 50 weight per cent and the plasticiser is contained in an amount of between 0.1 and 20 weight per cent.

12. A product obtained by the process of any one of Claims 1 to 11.

## Patentansprüche

1. Verfahren zur Herstellung poröser Mikrosphären mit einem Durchmesser zwischen 3 und 300 Mikrometern, umfassend:
(a) Herstellen einer organischen Phase, die eine Lösung aus einem Polymer und einem Plastifizierer aufweist, der aus der Gruppe ausgewählt ist, die aus Phthalatestern, Phosphatestern, Zitratestern, Sebacatestern, Glycerol, Triacetin und acetylierten Monoglyceriden besteht, in einem organischen Lösungsmittel;
(b) Herstellen einer wässrigen Phase, die eine wässrige Lösung aus einem oder mehreren Emulgatoren aufweist;
(c) Kombinieren der organischen und wässrigen Phasen unter emulgierenden Bedingungen, um eine Emulsion der organischen Phase in der wässrigen Phase zu bilden; und
(d) Verdampfen des Lösungsmittels, um Mikrosphären zu bilden.

2. Verfahren nach Anspruch 1, bei dem die Mikrosphären des Weiteren einen zu verabreichenden Bestandteil enthalten, wobei der zu verabreichende Bestandteil in der organischen Phase enthalten ist, die im Schritt (a) hergestellt wird.

3. Verfahren nach Anspruch 2, bei dem der zu verabreichende Bestandteil eine landwirtschaftliche Chemikalie, ein Pharmazeutikum, eine Medizin, ein Kosmetikum oder ein Duft ist.

4. Verfahren nach Anspruch 3, bei dem der zu verabreichende Bestandteil eine landwirtschaftliche Chemikalie ist.

5. Verfahren nach Anspruch 2, wobei der zu verabreichende Bestandteil ein Fungizid oder ein Bakterizid ist.

6. Verfahren nach einem der Ansprüche 1 bis 5, bei dem das Polymer ein Zellulosepolymer oder ein Derivat hiervon ist.

7. Verfahren nach einem der Ansprüche 1 bis 6, bei dem der Plastifizierer ein Phthalatester ist.

8. Verfahren nach einem der Ansprüche 1 bis 7, bei dem der Emulgator Polyvinylalkohol aufweist.

9. Verfahren nach einem der Ansprüche 1 bis 8, bei dem das Polymer in der organischen Phase in einer Menge von 1 bis 50 Gewichtsprozent vorliegt und der Plastifizierer in der organischen Phase in einer Menge von 0,1 und 20 Gewichtsprozent relativ zu dem Polymer vorliegt.

10. Verfahren nach einem der Ansprüche 2 bis 9, bei dem die Mikrosphären bis zu 70 Gewichtsprozent des zu verabreichenden Bestandteils enthalten.

11. Verfahren nach einem der Ansprüche 1 bis 10, bei dem das Polymer in der organischen Phase in einer Menge von zwischen 1 und 50 Gewichtsprozent enthalten ist und der Plastifizierer in einer Menge von zwischen 0,1 und 20 Gewichtsprozent enthalten ist.

12. Erzeugnis, das durch das Verfahren nach einem der Ansprüche 1 bis 11 erhalten ist.

## Revendications

1. Procédé pour la production de microsphères poreuses ayant un diamètre entre 3 et 300 microns, comprenant :
(a) la préparation d'une phase organique comprenant une solution d'un polymère et d'un plastifiant choisi dans le groupe consistant en phtalate esters, phosphate esters, citrate esters, sébacate esters, glycérol, triacétine et monoglycérides acétylés, dans un solvant organique ;
(b) la préparation d'une phase aqueuse Comprenant une solution aqueuse d'un ou de plusieurs agents émulsifiants;
(c) la combinaison des phases organique et aqueuse dans des conditions émulsifiantes pour former une émulsion de la phase organique dans la phase aqueuse ; et
(d) l'évaporation du solvant pour former des microsphères.

2. Procédé selon la revendication 1, dans lequel les microsphères contiennent de plus un ingrédient à dispenser, ledit ingrédient à dispenser étant contenu dans la phase organique préparée à l'étape (a).

3. Procédé selon la revendication 2, dans lequel l'ingrédient à dispenser est un produit chimique agricole, un produit pharmaceutique, un médicament, un cosmétique ou un parfum.

4. Procédé selon la revendication 3, dans lequel l'ingrédient à dispenser est un produit chimique agricole.

5. Procédé selon la revendication 2, dans lequel l'ingrédient à dispenser est un fongicide ou un bactéricide.

6. Procédé selon l'une quelconque des revendications 1 à 5, dans lequel le polymère est un polymère cellulosique ou son dérivé.

7. Procédé selon l'une quelconque des revendications 1 à 6, dans lequel le plastifiant est un phtalate ester.

8. Procédé selon l'une quelconque des revendications 1 à 7, dans lequel l'agent émulsifiant comprend de l'alcool de polyvinyle.

9. Procédé selon l'une quelconque des revendications 1 à 8, dans lequel le polymère est présent dans la phase organique dans une quantité de 1 à 50 % en poids et le plastifiant est présent dans la phase organique dans une quantité de 0,1 à 20 % en poids par rapport au polymère.

10. Procédé selon l'une quelconque des revendications 2 à 9, dans lequel les microsphères contiennent jusqu'à 70 % en poids de l'ingrédient à dispenser.

11. Procédé selon l'une quelconque des revendications 1 à 10, dans lequel le polymère est contenu dans la phase organique dans une quantité d'entre 1 et 50 % en poids et le plastifiant est contenu dans une quantité d'entre 0,1 et 20 % en poids.

12. Produit obtenu par le procédé de l'une quelconque des revendications 1 à 11.
